# EUROPÄISCHE PATENTSCHRIFT

(11) **EP 0 720 447 B1**
(45) Veröffentlichungstag und Bekanntmachung des Hinweises auf die Patenterteilung: **03.12.1997**
(21) Anmeldenummer: 94928313.9
(22) Anmeldetag: 03.09.1994
(51) Int. Cl.: A61B 17/06

(54) **ATRAUMATISCHE NADEL FÜR CHIRURGISCHE NÄHMASCHINEN**
ATRAUMATIC NEEDLE FOR SUTURE-INSERTION MACHINES
AIGUILLE ATRAUMATIQUE POUR APPAREILS CHIRURGICAUX DE SUTURE

(30) Priorität: 01.10.1993 DE 4333545
(43) Veröffentlichungstag der Anmeldung: 10.07.1996
(73) Patentinhaber: Moll, Clemens, D-52076 Aachen (DE)
(72) Erfinder: Moll, Clemens, D-52076 Aachen (DE)
(74) Vertreter: Klein, Friedrich
(86) Internationale Anmeldenummer: EP9402945
(87) Internationale Veröffentlichungsnummer: WO9509568

(56) Entgegenhaltungen:
- DE-A- 3 702 015
- GB-A- 559 222

## Beschreibung

Die Erfindung betrifft eine atraumatische Nadel zur Verwendung in chirurgischen Nähmaschinen nach dem Oberbegriff des Patentanspruches 1, dem die DE-A-37 02 015 zu Grunde liegt.

Das von Hand erfolgende Herstellen von Nähten in Gewebe sowohl der Human- als auch der Veterinärmedizin erfordert einerseits eine große Geschicklichtkeit und ist je nach Struktur des Gewebes nur unter Anwendung relativ hoher Kräfte durch den Operateur möglich. Zur Reduzierung der aufzuwendenden Durchstechkräfte werden u. a. Nadeln mit einer sog. Schneidspitze verwendet, die beispielsweise die Form einer Dreieckspitze aufweisen (siehe DE-OS 37 12 163).

Chirurgische Nähmaschinen, wie sie beispielsweise in der DE-PS 37 02 015 beschrieben sind, sind zwar in der Lage die unter Verwendung von mit einer kegelförmig ausgebildeten Spitze auftretenden Durchstechkräfte aufzubringen, jedoch wird hier vor dem eigentlichen Einstechen der Nadel eine große Kraft und damit ein relativ großer Druck auf die Gewebeflächen ausgeübt, die zu Verformungen des Randbereiches des jeweiligen Stichkanals führt. Solche Verformungen wirken sich zwar nur im Bereich von einigen Zehntel-Millimetern aus, sie reduzieren aber dennoch insoweit die Sicherheit der Nahtbildung, als die vom Greifer der chirurgischen Nähmaschine zu erfassende Schlinge des Fadens nicht ausreichend oder - bezogen auf die Bewegung des Greifers - nicht in der richtigen Lage oder nicht zum richtigen Zeitpunkt der Bewegung des Greifers ausgebildet wird. Da die Bildung von sog. Fehlstichen insbesondere im humanmedizinischen Bereich zu für den Patienten sehr schwer wiegenden Folgen führen kann, Ist es erforderlich, schon beim Einstechen der Nadel in das Gewebe sicherzustellen, daß die hierbei entstehenden Einstichkräfte keinen ungünstigen Einfluß auf die sich anschließende Stichbildung ausüben.

Der Erfindung liegt daher die Aufgabe zugrunde, eine Nadel für chirurgische Nähmaschinen zu schaffen, die bei ihrem Einstich in das Gewebe einerseits keinen die Sicherheit der Stich- insbesondere der Schlingenbildung beeinträchtigenden Einfluß auf das Gewebe ausübt und andererseits den Stichkanal nicht über das unbedingt erforderliche Maß aufweitet.

Diese Aufgabe wird erfindungsgemäß durch die im kennzeichnenden Teil des Patentanspruchs 1 angegebenen Maßnahmen gelöst. Durch die erfindungsgemäße Ausbildung entstehen einerseits aufgrund der gleichen Größe der Krümmungsradien sowohl des Schaftes als auch dessen Bewegungsbahn keine Verformungen am Randbereich des Stichkanals, andererseits werden durch die an der Außenfläche des Schaftes angeordnete spatenförmig ausgebildete Schneide, die durch bzw. während der Bildung des Stichkanals entstehenden Seitenkräfte erheblich reduziert und wirken stets nur in eine, nämlich zur Außenfläche des Schaftes gerichtete Richtung. Hierdurch werden einerseits Verformungen im Randbereich des Stichkanals vermieden und andererseits wird der Schaft der Nadel stets nach außen gedrückt, so daß für die Bildung der Fadenschlinge stets die gleichen Reibungskräfte und auch die gleichen Reibungsverhältnisse herrschen und die Fadenschlinge daher stets gleichmäßig ausgebildet und vom Greifer sicher erfaßt werden kann. Eine weitergehende Reduzierung der erforderlichen Einstichkraft sowie ein weitergehende Schonung des Randbereich des Stichkanals ergibt sich dadurch, daß die Schneide gemäß den Merkmalen des Patentanspruches 2 weitergebildet ist.

Eine weitere Verbesserung der Einstichverhältnisse ergibt sich durch die Merkmale des Patentanspruches 3.

Eine in konstruktiver Hinsicht einfache Anpassung des Krümmungsradius des Schaftes an den Krümmungsradius seiner Bewegungsbahn ist mit den Merkmalen des Patentanspruches 4 zu erreichen, wonach zwischen dem Kolben und dem Schaft der Nadel ein Zwischenstück vorgesehen ist, und dadurch der Krümmungsradius des Schaftes vergrößert wird.

Eine besonders sichere Erfassung der Fadenschlinge durch den Greifer ist dann gegeben, wenn dessen schlingenerfassende Spitze die Fadenschlinge möglichst dicht am Nadelöhr erfassen kann.

Um dies zu gewährleisten weist der Schaft an seiner dem Greifer der Nähmaschine zugewandten Seite im Bereich des Öhrs eine Ausnehmung für den Greifer auf.

Eine sichere Führung des Fadens zwischen dem der Nadel unmittelbar vorgeordneten Führungsmittel der Nähmaschine und dem Öhr der Nadel sowie eine Reduzierung der während des Einstiches der Nadel in das Gewebe entstehenden Reibungskräfte zwischen diesen und der Nadel ergibt sich dadurch, daß der Schaft an seiner der Ausnehmung für den Greifer gegenüberliegenden Seite eine Fadenführung aufweist. Diese Fadenführung kann entweder von einer im Bereich des Öhrs endenden Nut oder einer im Bereich des Öhrs auslaufenden Bohrung gebildet sein.

Die Erfindung wird anhand eines in der beigefügten Zeichnung dargestellten Ausführungsbeispiels näher erläutert. Es zeigen:
- Fig. 1:: eine Vorderansicht einer atraumatischen Nadel in vergrößertem Maßstab
- Fig. 2:: einen Längsschnitt durch die Nadel gemäß Fig. 1
- Fig. 3:: einen Schnitt nach Linie III-III der Fig. 2
- Fig. 4:: eine gegenüber Fig. 1 nochmals vergrößerte Darstellung des Bereiches der Spitze der Nadel
- Fig. 5:: eine gegenüber im Längsschnitt gemäß Fig. 2 nochmals vergrößerte Darstellung des Bereiches der Spitze der Nadel
- Fig. 6:: ein Schnitt nach Linie VI-VI der Fig. 5
- Fig. 7:: ein Schnitt nach Linie VII-VII der Fig. 5

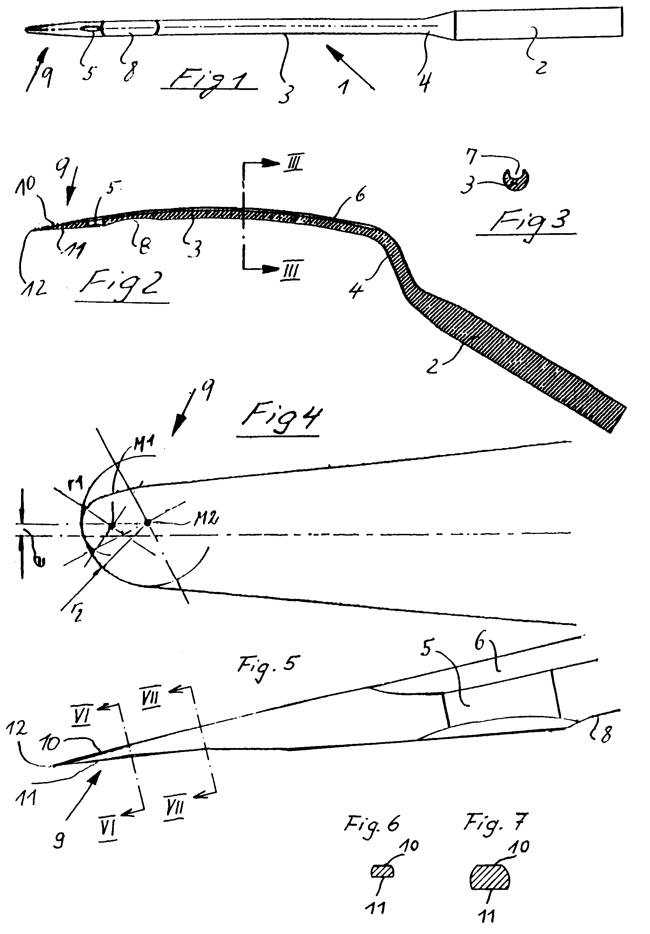

Die in Fig. 1 in Vorderansicht dargestellte atraumatische Nadel weist einen im wesentlichen zylinderförmig ausgebildeten Kolben 2 auf, der in einem Halter einer chirurgischen Nähmaschine, wie sie beispielsweise in der DE-PS 37 02 015 dargestellt ist, aufgenommen wird. Entsprechend der getriebemäßigen Ausbildung einer solchen chirurgischen Nähmaschine bewegt sich die Nadel auf einer kreisförmigen Bewegungsbahn.

Zwischen dem Kolben 2 und dem eigentlichen Schaft 3 der Nadel ist ein Zwischenstück 4 angeordnet, das ebenfalls einen zylindrischen Querschnitt aufweisen kann, dessen Durchmesser kleiner als der Durchmesser des Kolbens 2 ist. Das Zwischenstück 4 verläuft (siehe Fig. 2) sowohl zum Kolben 2 als auch zum Schaft 3 in einem bestimmten Winkel, wobei die Übergangsstellen sowohl vom Kolben 2 zum Zwischenstück 4 als auch von diesem zum Schaft 3 gerundet sind. Der Schaft 3 ist bogenförmig gestaltet, wobei sein Krümmungsradius dem Krümmungsradius seiner Bewegungsbahn bei in den Halter der Nähmaschine eingesetzter Nadel entspricht, so daß während des Eindringens der Nadelspitze in das Gewebe keine durch unterschiedliche Krümmungsradien von Schaft und Bewegungsbahn bedingten Relativbewegungen zwischen der Nadel und dem Gewebe entstehen und somit keine oder nur äußerst geringe Seitenkräfte auf das Gewebe ausgeübt werden und daher im Randbereich des Stichkanals keine Verletzungen des Gewebes entstehen können.

Im Bereich des dem Kolben 2 abgekehrten Endes der Nadel ist am Schaft 3 zur Aufnahme bzw. zum Durchtritt des zu vernähenden Fadens ein Öhr 5 vorgesehen. An der - bezogen auf Fig. 2 - Oberseite des Schaftes ist zwischen dessen Übergang zum Zwischenstück 4 und dem Öhr 5 eine Fadenführung 6 ausgebildet. Diese kann entsprechend Fig. 3 von einer Nut 7 gebildet sein, deren Öffnungsweite und -tiefe an die Dicke des zu vernähenden Fadens angepaßt sind. Die Nut 7 erstreckt sich parallel zur Oberseite des Schaftes vom Zwischenstück 4 bis zum Öhr 5. Die Fadenführung kann in einer weiteren, nicht besonders dargestellten Ausführungsform, von einer Bohrung entsprechenden Durchmessers gebildet sein, die sich ebenfalls vom Zwischenstück 4 bis zum Öhr 5 erstreckt und im wesentlichen parallel zum Oberseite des Schaftes 3 verläuft.

An der - ebenfalls bezogen auf Fig. 2 - Innenseite des Schaftes, die bei in den Halter der Nähmaschine eingesetzter Nadel die dem Greifer zugekehrte Seite des Schaftes bildet, ist - der Fadenführung 6 gegenüberliegend - eine Ausnehmung 8 für den Greifer der Nähmaschine vorgesehen, die einen geringen seitlichen Abstand zwischen dem Schaft 3 und dem Greifer und somit ein sicheres Erfassen der Fadenschlinge ermöglicht.

Die sich an das Öhr 5 anschließende Spitze 9 der Nadel weist einen zylinder- oder kegelförmigem Querschnitt auf, an dessen Außenseite eine Abflachung 10 vorgesehen ist. Zur Bildung einer im wesentlichen quer zur Längsachse der Nadel gerichteten Schneide 12 ist auch an der Innenseite der Spitze 9 eine Abflachung 11 vorgesehen, wobei beide Abflachungen 10, 11 im spitzen Winkel zueinander verlaufen und ihre Schnittgerade im wesentlichen auf der Außenseite der Spitze 9 liegt. Die hierdurch gebildete spatenförmige Schneide 12 liegt damit auf dem äußeren Krümmungsradius der Spitze 9 und somit außermittig zur Längsmittelachse der Nadel bzw. des Schaftes.

Bei dem in der Zeichnung dargestellten Ausführungsbeispiel der Erfindung sind die beiden Endbereiche der Schneide 12 gerundet, wobei die Rundungen von zwei Kreisbogen unterschiedlicher Radien R1 und R2 gebildet sind. Beide Mittelpunkte M1 und M2 der Kreisbogen liegen um einen Betrag "e" seitlich der Längsachse der Nadel und somit in einer zu deren Längsmittelebene im wesentlichen parallel gerichteten Ebene, die zur Längsmittelebene der Nadel den Abstand "e" aufweist. Hierdurch wird eine weitergehende Reduzierung sowohl der Einstichkräfte als auch von Verformungen im Randbereich des Stichkanals erreicht.

## Patentansprüche

1. Atraumatische Nadel zur Verwendung in chirurgischen Näh-maschinen, die einen Kolben zu ihrer Aufnahme in einem sich auf einer kreisbogenförmigen Bahn bewegenden Nadelhalter sowie einen im wesentlichen zylinderförmig ausgebildeten und gekrümmt verlaufenden Schaft mit einem Öhr zum Durchtritt des Fadens, sowie eine sich an das Öhr anschließende Spitze aufweist, dadurch gekennzeichnet,
daß der Krümmungsradius des Schaftes (3) im wesentlichen dem Krümmungsradius seiner Bewegungsbahn entspricht und im Bereich der Spitze (9) sowohl an der **radialen** Außen- als auch an der **radialen** Innenfläche des Schaftes (3) je eine Abflachung (10,11) vorgesehen ist, wobei beide Abflachungen (10,11) im spitzen Winkel zueinander verlaufen und ihre Schnittlinie zur Bildung einer zur Längsachse des Schaftes (3) quer gerichteten und im wesentlichen auf dessen Außenseite liegenden Schneide (12) **im wesentlichen** auf dem äußeren Krümmungsradius des Schaftes (3) liegt.

2. Atraumatische Nadel nach Anspruch 1, dadurch gekennzeichnet, daß die Schneide (12) unsymmetrisch zur durch das Öhr (5) verlaufenden Längsachse des Schaftes (3) liegt und gerundet ist.

3. Atraumatische Nadel nach Anspruch 2, dadurch gekennzeichnet, daß die Schneide (12) von zwei Kreisbögen mit unterschiedlichen Radien (r1,r2) gebildet ist, deren Mittelpunkte (M1,M2) in einer zur Längsmittelebene der Nadel im wesentlichen parallel gerichteten Ebene liegen.

4. Atraumatische Nadel nach Anspruch 1, dadurch gekennzeichnet, daß zwischen dem Kolben (2) und dem Schaft (3) ein zu diesem winklig verlaufendes Zwischenstück (4) angeordnet ist, wobei der Kolben (2) seinerseits winklig zum Zwischenstück (4) verläuft.

5. Atraumatische Nadel nach Anspruch 1, dadurch gekennzeichnet, daß der Schaft (3) an seiner dem Greifer zugewandten Seite im Bereich des Öhrs (5) eine Ausnehmung (8) für den Greifer aufweist.

6. Atraumatische Nadel nach Anspruch 5, dadurch gekennzeichnet, daß der Schaft an seiner der Ausnehmung (8) gegenüberliegenden Seite eine Fadenführung (6) aufweist.

7. Atraumatische Nadel nach Anspruch 6, dadurch gekennzeichnet, daß die Fadenführung (6) von einer im Bereich des Öhrs (5) endenden Nut (7) gebildet ist.

8. Atraumatische Nadel nach Anspruch 1 und 6, dadurch gekennzeichnet, daß die Fadenführung (6) von einer im Bereich des Öhrs (5) auslaufenden Bohrung gebildet ist.

## Claims

1. Atraumatic needle for use in surgical sewing machines, said needle comprising a butt for its accommodation in a needle holder which moves along an arcuate path, as well as a shaft, which has a substantially cylindrical configuration and extends in a curved manner, said shaft being provided with an eye for the thread to pass therethrough, and a point which communicates with the eye, characterised in that the radius of curvature of the shaft (3) corresponds substantially to the radius of curvature of its path of movement, and a respective flattened portion (10, 11) is provided in the region of the point (9), both on the radial outer surface and on the radial inner surface of the shaft (3), both flattened portions (10, 11) extending at an acute angle relative to each other, and their line of intersection lying substantially on the outer radius of curvature of the shaft (3) to form a cutting edge (12), which is orientated transversely relative to the longitudinal axis of the shaft (3) and lies substantially on the outer surface of said shaft.

2. Atraumatic needle according to claim 1, characterised in that the cutting edge (12) lies unsymmetrically relative to the longitudinal axis of the shaft (3), which axis extends through the eye (5), and is rounded.

3. Atraumatic needle according to claim 2, characterised in that the cutting edge (12) is formed from two arcs with different radii (r1, r2), the centres (M1, M2) of which lie in a plane orientated substantially parallel to the central longitudinal plane of the needle.

4. Atraumatic needle according to claim 1, characterised in that an intermediate part (4) is disposed between the butt (2) and the shaft (3) extends angularly relative to the shaft, the butt (2), for its part, extending angularly relative to the intermediate part (4).

5. Atraumatic needle according to claim 1, characterised in that the shaft (3) comprises a recess (8) for the gripper on its side flattened portion the gripper in the region of the eye (5).

6. Atraumatic needle according to claim 5, characterised in that the shaft comprises a thread guide (6) on its side lying opposite the recess (8).

7. Atraumatic needle according to claim 6, characterised in that the thread guide (6) is formed by a groove (7) ending in the region of the eye (5).

8. Atraumatic needle according to claims 1 and 6, characterised in that the thread guide (6) is formed by a bore terminating in the region of the eye (5).

## Revendications

1. Aiguille atraumatique à utiliser sur des machines pour suture chirurgicale, qui comprend un piston permettant de loger ladite aiguille dans un porte-aiguille qui se déplace selon une trajectoire en forme d'arc de cercle, ainsi qu'une tige de forme sensiblement cylindrique et d'allure courbe munie d'un chas, dans lequel passe le fil, ainsi qu'une pointe qui prolonge la partie avec chas, caractérisée en ce que le rayon de courbure de la tige (3) coïncide sensiblement avec le rayon de courbure de sa trajectoire de déplacement et il est prévu, dans la zone de la pointe (9), de réaliser un aplatissement (10, 11), sur la face radiale extérieure et sur la face radiale intérieure de la tige (3), les deux aplatissements (10, 11) formant l'un avec l'autre un angle aigu et leur ligne d'intersection étant située sensiblement sur le rayon de courbure extérieur de la tige (3) pour former un bord effilé (12) orienté transversalement par rapport a' l'axe longitudinal de la tige (3) et sensiblement situé sur le côté extérieur' de cette dernière.

2. Aiguille atraumatique selon la revendication 1, caractérisée en ce que le bord effilé (12) est asymétrique par rapport à l'axe longitudinal de la tige (3) qui traverse le chas (5) et ledit bord effilé est arrondi.

3. Aiguille atraumatique selon la revendication 2, caractérisée en ce que le bord effilé (12) est formé par deux arcs de cercle avec des rayons différents (r1, r2), dont les centres (M1, M2) sont situés dans un plan sensiblement parallèle au plan médian longitudinal de l'aiguille.

4. Aiguille atraumatique selon la revendication 1, caractérisée en ce qu'une pièce intermédiaire (4) est située entre le piston (2) et la tige, (3) en formant un angle avec cette dernière, le piston (2) formant lui-même un angle avec la pièce intermédiaire (4).

5. Aiguille atraumatique selon la revendication 1, caractérisée en ce que la tige (3), sur le côté orienté vers le crochet, présente dans la zone du chas (5) un évidement (8) pour le crochet.

6. Aiguille atraumatique selon la revendication 5, caractérisée en ce que la tige (3) est munie d'un guide-fil (6) sur son côté opposé à l'évidement (8).

7. Aiguille atraumatique selon la revendication 6, caractérisée en ce que le guide-fil (6) est formé par une rainure (7) qui se termine dans la zone du chas (5).

8. Aiguille atraumatique selon les revendications 1 et 6, caractérisée en ce que le guide-fil (6) est formé par une forure qui s'étend dans la zone du chas (5).
